(19) **Europäisches Patentamt**

**European Patent Office**

**Office européen des brevets**

(11) Veröffentlichungsnummer: **0 602 128 B1**

(12) ## EUROPÄISCHE PATENTSCHRIFT

(45) Veröffentlichungstag der Patentschrift: **06.12.95**

(51) Int. Cl.6: **A61K 6/10**, C08L 83/04

(21) Anmeldenummer: **92918766.4**

(22) Anmeldetag: **03.09.92**

(86) Internationale Anmeldenummer:
**PCT/EP92/02036**

(87) Internationale Veröffentlichungsnummer:
**WO 93/04659 (18.03.93 93/08)**

Die Akte enthält technische Angaben, die nach
dem Eingang der Anmeldung eingereicht wurden
und die nicht in dieser Patentschrift enthalten sind.

(54) **LAGERSTABILE, PERMANENT WASSERBENETZBARE VULKANISATE ERGEBENDE POLYSILOXANMASSE.**

(30) Priorität: **06.09.91 DE 4129613**

(43) Veröffentlichungstag der Anmeldung:
**22.06.94 Patentblatt 94/25**

(45) Bekanntmachung des Hinweises auf die
Patenterteilung:
**06.12.95 Patentblatt 95/49**

(84) Benannte Vertragsstaaten:
**AT BE CH DE ES FR GB IT LI NL SE**

(56) Entgegenhaltungen:
**EP-A- 0 231 420**
**EP-A- 0 398 745**
**EP-A- 0 429 069**
**DE-A- 2 922 295**
**US-A- 4 657 959**

(73) Patentinhaber: **WACKER-CHEMIE GMBH**
**Hanns-Seidel-Platz 4**
**D-81737 München (DE)**

(72) Erfinder: **STEPP, Michael**
**Mehringer Strasse 4**
**D-8263 Burghausen (DE)**
Erfinder: **HEFNER, Heinz**
**Vivaldistr. 2**
**D-8263 Burghausen (DE)**
Erfinder: **HUBER, Peter**
**Gluckstr. 39**
**D-8263 Burghausen (DE)**
Erfinder: **MÜLLER, Johann**
**Friedrich-Eberth-Strasse 3**
**D-8263 Burghausen (DE)**
Erfinder: **SCHMIDLKOFER, Richard**
**Augenthalerweg 26**
**D-8263 Burghausen (DE)**

Ullmanns Encyklopädie der technischen Chemie, 4. Auflage, Band 18, Verlag Chemie, Weinheim 1979, Seite 717

Erfinder: **GARHAMMER, Arnold**
**Mühlenweg 12**
**D-8265 Simbach (DE)**

**Beschreibung**

Die vorliegende Erfindung betrifft lagerstabile Polysiloxanmassen, die nach dem Vulkanisieren permanent wasserbenetzbare Elastomere ergeben, ein Verfahren zu deren Herstellung und deren Verwendung als dentale Abformmassen.

Polysiloxanmassen, die zu Elastomeren vulkanisieren finden breite Anwendung als Abformmassen. Dabei haben sich die additionsvernetzenden Systeme besonders bewährt, da sie schneller abbinden und im Gegensatz zu den kondensationsvernetzenden Systemen keine großen Mengen an problematischem Katalysator benötigen.

Ein wesentlicher Nachteil von Siliconabformpräparaten auf der Basis hydrophober Polysiloxane beruht auf der Tatsache, daß eine präzise Abformung feuchter Oberflächen, wie Gewebe-, Zahn- oder Zahnersatzoberflächen in der Mundhöhle kaum möglich ist, da die Feuchtigkeitsanteile durch die große Oberflächenspannung des Wassers zu einer Form mit der geringsten Oberfläche streben. Die Feuchtigkeit setzt sich deshalb tropfenförmig zwischen die abzuformende Oberfläche und die Abformmasse.

Eine oberflächenaktive Abformmasse bewirkt, daß die Feuchtigkeitsanteile auf den abzuformenden Oberflächen zu einem dünnen Feuchtigkeitsfilm spreiten. Dadurch werden Hohlräume im Abdruck vermieden und eine präzise Wiedergabe der Oberflächenstruktur erreicht.

Die Herstellung von wasserbenetzbaren Siliconabformmassen durch Vermischen der Polysiloxanmassen mit relativ niedermolekularen oberflächenaktiven Agentien ist bekannt: In K.B. Norling, M.H. Reisbick; The Journal of Prosthetic Dentistry 42, 342-347 (1979) ist die Verwendung von Nonylphenoxypoly(ethylenoxy)-ethanol, in der DE-A-37 21 784 (S.Futami und S.Terauchi; offengelegt am 21.1.1988 für G-C Dental Industrial Corp., Tokyo, JP) ist die Verwendung von Proteinen, hydrophilen Siliconölen und nichtionischen grenzflächenaktiven Mitteln, und in der EP-A-231 420 (H.Gribi; offengelegt am 12.8.1987 für Dentsply GmbH, Konstanz, DE) ist die Verwendung von Siloxankomponenten mit Alkylenethergruppen in Polysiloxanmassen beschrieben.

Die vorstehenden Abformmassen weisen den Nachteil auf, daß die oberflächenaktiven Agentien teilweise eine Entmischungstendenz gegenüber den Abformmassen aufweisen, an deren Oberfläche wandern oder durch die schlechte Bindung in den Abformmassen in Kontakt mit wässrigen Medien extrahiert werden. Im letzteren Fall geht die Wasserbenetzbarkeit durch beispielsweise Abspülen mit Wasser, Desinfektion, Sterilisation, Erstellen von Duplikaten mit Hilfe wasserhaltiger Abformpräparate, wie Gips verloren, bevor die angestrebten anwendungstechnischen Ziele, wie reproduzierbare Benetzungseigenschaften des Siliconabdrucks nach der Entformung von der feuchten Oberfläche erreicht sind.

Aus der DE-A 1 519 412 (E.L. Morehouse; offengelegt am 21.5.1970 für Union Carbide Corp., New York, U.S.A.) sind in Wasser oder in einem wasserlöslichen organischen Lösungsmittel gelöste Organosiloxan-Alkylenether-Mischpolymere als Antibeschlagmittel bekannt, wobei die Mischpolymere aliphatische Doppelbindungen oder Si-H-Gruppen aufweisen können.

Gemäß dem dort beschriebenen Herstellungsverfahren werden Allylendgruppen aufweisende Alkylenethergruppen in einer Hydrosilylierungsreaktion mit Dichlormethylsilan in Gegenwart eines Platinkatalysators umgesetzt. Das Produkt wird dann mit Vinylgruppen-, Si-H-Gruppen-, Chlor- und Methylgruppen-haltigen Silanen zu den Mischpolymeren hydrolysiert.

In der EP-A-231 420 wird für Siliconabformmassen u.a. die Bindung von Polyethereinheiten an eine der Komponenten der Polysiloxangrundmasse vorgeschlagen. Durch die Einvernetzung der Polyethereinheiten können permanent wasserbenetzbare Vulkanisate erhalten werden.

Jedoch weisen die in der EP-A-231 420 beschriebenen, die Benetzbarkeit verbessernden, mit der Polysiloxanmasse mischbaren Siloxankomponenten mit Alkylenethergruppen und die in der DE-A-1 519 412 beschriebenen, als hydrophile Modifier in die Polysiloxanmasse einvernetzbaren Polysiloxane mit Alkylenethereinheiten herstellungsbedingt einen Gehalt an Edelmetallkatalysator auf. Zusätzlich weisen die bisher herstellbaren Doppelbindungen enthaltenden hydrophilen Modifier geringe Mengen an Si-H Gruppen auf und die bisher herstellbaren Si-H Gruppen enthaltenden hydrophilen Modifier geringe Mengen an Doppelbindungen.

In der EP-A-429 069 ist eine Siliconkautschukzusammensetzung beschrieben, die Alkenylgruppen aufweisendes Organopolysiloxan, Si-H Gruppen aufweisendes Organopolysiloxan und einvernetzbare Siloxane mit Alkenylgruppen und Alkylenethereinheiten aufweist.

Die EP-A-398 745 beschreibt dentale Siliconkautschukabformmassen, die Alkenylgruppen aufweisendes Organopolysiloxan, Si-H Gruppen aufweisendes Organopolysiloxan und einvernetzbare Siloxane mit Alkylenethereinheiten und Alkenylgruppen oder Si-H Gruppen aufweist.

Bei der Verwendung als hydrophile Modifier ergeben die bekannten Mischpolymere aufgrund der vorstehend beschriebenen Mängel weder mit der Zweikomponentenkautschuk-Komponente (A) die eine Polysiloxanmasse mit aliphatischen Doppelbindungen und einen Edelmetallkatalysator umfaßt, noch mit der Komponente (B), die eine Polysiloxanmasse mit Si-H-Gruppen umfaßt, lagerstabile Gemische. In allen Fällen entstehen bei der Lagerung Vulkanisatanteile.

Die getrennte Lagerung des hydrophilen Modifiers ist kein befriedigender Ausweg, da eine Dreikomponentenpolysiloxanmasse bei der Anwendung in der Praxis zu schlecht handhabbar ist.

Der vorliegenden Erfindung liegt die Aufgabe zugrunde, permanent wasserbenetzbare elastomere Vulkanisate ergebende Polysiloxanmassen bereitzustellen, deren zwei Komponenten bei der Lagerung gegenüber einer vorzeitigen Vulkanisation stabil sind.

Gegenstand der Erfindung ist eine lagerstabile, permanent wasserbenetzbare elastomere Vulkanisate ergebende Polysiloxanmasse, bestehend aus den getrennt gelagerten Komponenten

(A), die eine Masse aus Polysiloxanen, welche SiC-gebundene $C_1$-$C_6$-Alkylreste und/oder Phenylreste aufweisen und mindestens 2 $C_1$-$C_6$-Alkenylreste pro Molekül besitzen, und einen Edelmetallkatalysator umfaßt, wobei die Komponente (A) im wesentlichen frei von Si-H-Gruppen ist und

(B), die eine edelmetallkatalysatorfreie Masse aus Polysiloxanen, welche SiC-gebundene $C_1$-$C_6$-Alkylreste und/oder Phenylreste aufweisen und mindestens 3 Si-H-Gruppen pro Molekül besitzen, und gegebenenfalls zusätzlich aus Polysiloxanen, welche SiC-gebundene $C_1$-$C_6$-Alkylreste und/oder Phenylreste aufweisen und mindestens 2 $C_1$-$C_6$-Alkenylreste pro Molekül besitzen umfaßt, wobei ein hydrophiler Modifier der allgemeinen Formel I

$$[R_3SiO_{1/2}]_a[R^1R_2SiO_{1/2}]_b[ZR_2SiO_{1/2}]_c[ZRR^1SiO_{1/2}]_d \qquad [RR^1SiO_{2/2}]_e[R_2SiO_{2/2}]_f[SiO_{4/2}]_g[RSiO_{3/2}]_h-[R^1SiO_{3/2}]_i, \qquad (I)$$

in der die Reste
R Wasserstoffatome oder gleiche oder verschiedene einwertige, gegebenenfalls halogensubstituierte über SiC-gebundene $C_1$-$C_{12}$-Kohlenwasserstoffreste bedeuten, wobei mindestens einer der Reste R eine aliphatische Doppelbindung aufweist oder ein Wasserstoffatom bedeutet;
$R^1$ die allgemeine Formel II

$$E[OY]_xR^2 \qquad (II)$$

bedeutet, in der E eine Einfachbindung oder einen $C_1$-$C_6$-Alkylenrest, Y gleiche oder verschiedene $C_1$-$C_4$-Alkylenreste bedeutet, $R^2$ eine Hydroxylgruppe, einen $C_1$-$C_6$-Alkoxy- oder $C_1$-$C_6$-Oxycarbonylalkylrest bedeutet und x die Werte 1 bis 20 hat;
Z die Bedeutungen von $R^2$ aufweist oder ein Halogenatom bedeutet;
mit der Maßgabe, daß a, b, c und d jeweils unabhängig voneinander Werte von 0 bis 8 haben, die Summe von a+b+c+d 2 bis 8 beträgt, die Summe von a+b+c+d+e+f+g+h+i 10 bis 400 beträgt und das Verhältnis der Summen a+c+f+g+h : b+d+e+i 100 : 1 bis 1 : 1 beträgt, welcher

i) Reste mit aliphatischen Doppelbindungen aufweist, in der Komponente (A) und (B) enthalten ist und/oder

ii) Si-H-Gruppen und/oder Reste mit aliphatischen Doppelbindungen aufweist, in der Komponente (B) enthalten ist,

mit der Maßgabe, daß wenn der hydrophile Modifier Si-H-Gruppen aufweist oder in eine Si-H-Gruppenhaltige Komponente (B) gemischt wird, c+d < 0,02
(a+b+c+d+e+f+g+h+i) ist,

**dadurch gekennzeichnet**, daß der hydrophile Modifier frei von Edelmetallkatalysator ist.

Bei den erfindungsgemäß verwendbaren Zweikomponentensystemen wird der zur Vulkanisation erforderliche Edelmetallkatalysator in die Polysiloxanmasse der Komponente (A) gemischt. Selbst wenn die Komponente (B) und die verwendete Katalysatorzubereitung keine aliphatischen Doppelbindungen enthalten kann keine befriedigend lagerstabile Polysiloxanmasse erhalten werden, wenn der Katalysator ebenfalls in Komponente (B) enthalten ist. Durch Feuchtigkeitsreste, die z.B. durch Füllstoffe oder Modifier eingebracht werden, entwickelt sich mit Si-H-Gruppen und Katalysator Wasserstoff.

Deshalb kann auch ein Si-H-Gruppen haltiger hydrophiler Modifier zur Lagerung nur in die Komponente (B) gemischt werden. Der erfindungsgemäß hergestellte hydrophile Si-H-Gruppen haltige Modifier ergibt eine lagerbeständige Komponente (B).

Der erfindungsgemäß hergestellte aliphatische Doppelbindungen enthaltende hydrophile Modifier kann auch in die Komponente (B) gemischt werden, da er frei von Edelmetallkatalysator ist. In diesem Fall stört ein Anteil von aliphatischen Doppelbindungen in der Komponente (B) nicht. Der hydrophile Modifier kann aber auch in die Komponente (A) gemischt werden, wenn er frei von Si-H-Gruppen ist. Mit dem aliphatische Doppelbindungen enthaltenden hydrophilen Modifier sind somit lagerbeständige Komponenten für die erfindungsgemäße Polysiloxanmasse herstellbar.

Die erfindungsgemäße lagerbeständige Polysiloxanmasse ist sowohl vor als auch nach dem Vulkanisieren sehr gut wasserbenetzbar. Die Benetzbarkeit der Vulkanisate bleibt auch nach längerem Kontakt mit wässrigen Systemen, beipielsweise nach Sterilisation praktisch unverändert.

Vorzugsweise weist der hydrophile Modifier ausschließlich Reste mit aliphatischen Doppelbindungen auf und ist sowohl in der Komponente (B) als auch in der Komponente (A) enthalten. Bei dieser Ausführungsform kann die Gesamtmenge an hydrophilem Modifier in der lagerstabilen Polysiloxanmasse und damit die hydrophilen Eigenschaften beträchtlich erhöht werden, ohne daß Entmischung eintritt.

Ein weiterer Vorteil dieser Ausführungsform liegt darin, daß der hydrophile Modifier in beiden Komponenten homogen und in gleicher Konzentration vorhanden sein kann. Dies ist bei der Anwendung moderner Applikationsformen, wie Doppelkammerkartuschen mit aufgesetztem Statikmischer durch die sehr kurzen Mischzeiten von großer Bedeutung.

Beispiele für Reste R sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest; Cycloalkylreste, wie Cyclopentyl-, Cyclohexyl-, Cycloheptylreste und Methylcyclohexylreste; Arylreste, wie der Phenyl- und der Naphthylrest; Alkarylreste, wie o-, m-, p-Tolylreste, Xylylreste und Ethylphenylreste; Aralkylreste, wie der Benzylrest, der alpha- und der $\beta$-Phenylethylrest.

Die vorstehenden Reste R enthalten gegebenenfalls eine aliphatische Doppelbindung. Beispiele sind Alkenylreste, wie der Vinyl-, Allyl-, 5-Hexen-1-yl-, E-4-Hexen-1-yl-, Z-4-Hexen-1-yl-, 2-(3-Cyclohexenyl)-ethyl- und Cyclododeca-4,8-dienylrest.

Beispiele für halogensubstituierte $C_1$-$C_{12}$-Kohlenwasserstoffreste sind mit Fluor-, Chlor-, Brom- und Jodatomen substituierte Alkylreste, wie der 3,3,3-Trifluor-n-propylrest, der 2,2,2,2',2',2'-Hexafluorisopropylrest, der Heptafluorisopropylrest, und Halogenarylreste, wie der o-, m-, und p-Chlorphenylrest.

Bevorzugte Reste R ohne aliphatische Doppelbindung sind der Methyl- und Phenylrest. Bevorzugte Reste R mit aliphatischer Doppelbindung sind der Vinyl-, Allyl-, und 5-Hexen-1-ylrest.

Die Alkylenreste sind zweiwertige gerad- oder verzweigtkettige Alkylreste, die über zwei Bindungen an Kohlenstoffatomen des Alkylrestes in den hydrophilen Modifier eingebunden sind.

Die Alkoxyreste sind über ein Sauerstoffatom gebundene gerad- oder verzweigtkettige Alkylreste.

Die Oxycarbonylalkylreste sind Carbonsäurereste mit einem gerad- oder verzweigtkettigen Alkylrest.

Die vorstehenden Beispiele für Alkylreste beziehen sich auch auf die vorstehend erläuterten Alkylen-, Alkoxy- und Oxycarbonylalkylreste.

Bevorzugte Alkylen-, Alkoxy- und Oxycarbonylalkylreste haben 1 bis 3 Kohlenstoffatome.

Große Werte von f und kleine Werte von e und x verbessern die Mischbarkeit von Polysiloxanmasse und hydrophilem Modifier. Der bevorzugte hydrophile Modifier vermischt sich gut mit den Komponenten (A) und (B) und verleiht der Polysiloxanmasse gute Wasserbenetzbarkeit, wenn das Verhältnis $a+c+f+g+h : x(b+d+e+i)$ 60:40 bis 90:10 beträgt. Vorzugsweise hat x Werte von 3 bis 10.

Die Mischbarkeit des hydrophilen Modifiers mit der Polysiloxanmasse ist besonders gut, wenn eine relativ geringe Verzweigung des Siloxangerüsts des Modifiers, d.h. $f > 0,8(f+g+h+i)$ und $e > 0,6(d+e+i)$ eingehalten wird, b, c, d, g, h und $i < 0,2(a+b+c+d+e+f+g+h+i)$, insbesondere $< 0,1-(a+b+c+d+e+f+g+h+i)$ und wenn vorzugsweise mehr als die Hälfte der Endgruppen im Siloxangerüst ausschließlich Reste R tragen sollen, d.h. $a > 0,5(a+b+c+d)$, insbesondere $a > 0,6(a+b+c+d)$.

Gut verarbeiten, d.h. schnell und homogen mit der Polysiloxanmasse vermischen läßt sich der hydrophile Modifier, wenn die Summe $a+b+c+d+e+f+g+h+i$ 10 bis 400, vorzugsweise 10 bis 220, insbesondere 20 bis 160 beträgt.

Herstellungsbedingt weist der hydrophile Modifier oft geringe Mengen Reste Z auf, die eine Hydroxylgruppe bedeuten. Wenn der hydrophile Modifier Si-H-Gruppen aufweist oder in eine Si-H-Gruppen haltige Komponente (B) gemischt wird muß in diesem Fall $c+d < 0,02(a+b+c+d+e+f+g+h+i)$ sein, um eine die Lagerstabilität beeinträchtigende Wasserstoffentwicklung gering zu halten. Die hydrolysierbaren Gruppen Z sind vorzugsweise in weniger als 10% der Siloxaneinheiten enthalten, d.h. $c+d < 0,1(a+b+c+d+e+f+g+h+i)$.

Die Komponente (A) umfaßt vorzugsweise eine Masse aus Polysiloxanen, die SiC-gebundene $C_1$-$C_6$-Alkylreste, insbesondere Methylreste und/oder Phenylreste aufweist und mindestens 2 $C_1$-$C_6$-Alkenylreste pro Molekül besitzt, die die aliphatischen Doppelbindungen enthalten. Die bevorzugten Alkenylreste sind Vinylreste und Allylreste. Vorzugsweise enthält ein Molekül nicht mehr als 10 Alkenylreste.

Die Kettenlänge der Polysiloxane der Komponente (A) soll vorzugsweise 2000 Si-Einheiten pro Molekül nicht überschreiten. Besonders bevorzugt ist Polydimethylsiloxan mit beidseitig endständigen Vinylgruppen mit einer Kettenlänge von 40 bis 1200 Si-Einheiten. Die Viskosität beträgt bei 25°C vorzugsweise 35 bis 100000 $mm^2/s$, insbesondere 500 bis 10000 $mm^2/s$.

Die Komponente (A) ist im wesentlichen frei von Si-H Gruppen, damit sie durch ihren Gehalt an Edelmetallkatalysator bei der Lagerung keine unerwünschten Vulkanisate bildet. Die Komponente (A) der lagerstabilen Polysiloxanmasse enthält als Edelmetallkatalysatoren zur Vulkanisation vorzugsweise Platinmetalle und/oder deren Verbindungen, vorzugsweise Platin und/oder dessen Verbindungen. Es können hier alle Katalysatoren eingesetzt werden, die auch bisher zur Addition von Si-H-Gruppen an aliphatisch ungesättigte Verbindungen eingesetzt wurden. Beispiele für solche Katalysatoren sind metallisches und feinverteiltes Platin, das sich auf Trägern, wie Siliciumdioxyd, Aluminiumoxyd oder Aktivkohle befinden kann, Verbindungen oder Komplexe von Platin, wie Platinhalogenide, z. B. $PtCl_4$, $H_2PtCl_6.6H_2O$, $Na_2PtCl_4$. $4H_2O$, Platin-Olefin-Komplexe, Platin-Alkohol-Komplexe, Platin-Alkoholat-Komplexe, Platin-Ether-Komplexe, Platin-Aldehyd-Komplexe, Platin-Keton-Komplexe, einschließlich Umsetzungsprodukten aus $H_2PtCl_6$ . $6H_2O$ und Cyclohexanon, Platin-Vinylsiloxankomplexe, insbesondere Platin-Divinyltetramethyldisiloxankomplexe mit oder ohne Gehalt an nachweisbarem anorganisch gebundenem Halogen, Bis-(gammapicolin)-platindichlorid, Trimethylendipyridinplatindichlorid, Dicyclopentadienplatindichlorid, Dimethylsulfoxydiethylenplatin-(II)-dichlorid sowie Umsetzungsprodukte von Platiptetrachlorid mit Olefin und primärem Amin oder sekundärem Amin oder primärem und sekundärem Amin, wie das Umsetzungsprodukt aus in 1-Octen gelöstem Platintetrachlorid mit sec.-Butylamin, oder Ammonium-Platinkomplexe gemäß EP-B 110 370.

Platinkatalysator wird vorzugsweise in Mengen 0,5 bis 500 Gewichts-ppm (Gewichtsteilen je Million Gewichtsteilen), insbesondere 2 bis 400 Gewichts-ppm, jeweils berechnet als elementares Platin und bezogen auf das Gesamtgewicht der in den Komponenten (A) und (B) vorliegenden Polysiloxanmasse, eingesetzt. Die meisten der oben genannten Platinkatalysatoren sind dermaßen aktiv, daß der Komponente (A) und/oder (B) ein Inhibitor zugesetzt werden muß, der die vorzeitige Vernetzung zum Elastomeren verhindert. Nicht notwendig ist eine solche Inhibition, falls beispielsweise Ammonium-Platinkomplexe gemäß EP-B 110 370 eingesetzt werden.

Inhibitoren sind bekannt und z. B. in US-A 3,933,880 beschrieben. Beispiele hierfür sind acetylenisch ungesättigte Alkohole, wie 3-Methyl-1-butin-3-ol, 1-Ethinylcyclohexan- 1-ol, 3,5-Dimethy1-1-hexin-3-ol und 3-Methyl-1-pentin-3-ol. Beispiele für Inhibitoren auf Vinylsiloxanbasis sind 1,1,3,3-Tetramethyl-1,3-divinylsiloxan und vinylgruppenhaltige Poly-, Oligo- und Disiloxane.

Die Komponente (B) umfaßt eine Masse aus Polysiloxanen, die vorzugsweise SiC-gebundene $C_1$-$C_6$-Alkylreste, insbesondere Methylreste und/oder Phenylreste aufweist und mindestens 3 Si-H-Gruppen pro Molekül besitzt. Vorzugsweise enthält ein Molekül nicht mehr als 5 Si-H-Gruppen.

Die Kettenlänge der Polysiloxane mit Si-H-Gruppen der Komponente (B) soll vorzugsweise 1000 Si-Einheiten pro Molekül nicht überschreiten. Die Viskosität beträgt bei 25°C vorzugsweise 20 bis 50000 $mm^2/s$, insbesondere 100 bis 5000 $mm^2/s$.

Die Komponente (B) enthält vorzugsweise zusätzlich Polysiloxane mit aliphatischen Doppelbindungen, die vorzugsweise die gleichen Eigenschaften aufweisen, wie die vorstehenden Polysiloxane der Komponente (A).

Sowohl die Komponente (A) als auch (B) kann neben den vorstehenden Bestandteilen Füllstoffe enthalten, wie nicht verstärkende Füllstoffe, also Füllstoffe mit einer BET-Oberfläche von bis zu 50 $m^2/g$, wie Quarz, Cristobalit, Diatomeenerde, Calciumsilikat, Zirkoniumsilikat, Montmorillonite, wie Bentonite, Zeolithe, einschließlich der Molekularsiebe, wie Natriumaluminiumsilikat, Metalloxidpulver, wie Aluminium-, oder Zinkoxide bzw. deren Mischoxide, Bariumsulfat, Calciumcarbonat, Gips, Glas- und Kunststoffpulver; verstärkende Füllstoffe, also Füllstoffe mit einer BET-Oberfläche von mehr als 50 $m^2/g$, wie pyrogen hergestellte Kieselsäure, gefällte Kieselsäure und Silicium-Aluminium-Mischoxide großer BET-Oberfläche. Die genannten Füllstoffe können hydrophobiert sein, beispielsweise durch die Behandlung mit Organosilanen bzw. -siloxanen oder durch Verätherung von Hydroxylgruppen zu Alkoxygruppen. Es kann eine Art von Füllstoff, es kann auch ein Gemisch von mindestens zwei Füllstoffen eingesetzt werden. Der Gesamtgehalt der beiden Komponenten an Füllstoffen beträgt vorzugsweise 10 bis 80 Gew.-%.

Sowohl die Komponente (A) als auch (B) kann zusätzlich Pigmente, wie Titandioxid oder Aluminiumspinelle, wie Kobaltaluminiumspinell enthalten. Der bevorzugte Gesamtgehalt beider Komponenten beträgt höchstens 10 Gew.-%.

Sowohl die Komponente (A) als auch (B) können für bestimmte Zwecke neben den vorstehenden Bestandteilen Zusatzstoffe enthalten. Geeignete Zusatzstoffe sind Fungizide, Bakterizide, Algicide, Mikrobicide, Geruchsstoffe, Geschmackstoffe Korrosionsinhibitoren, und, wenn auch nicht bevorzugt, organische Lösungsmittel enthalten. Die Komponenten (A) und (B) enthalten Zusatzstoffe jeweils vorzugsweise in Mengen von 0,001 bis 1 Gew.-%, insbesondere von 0,01 bis 0,1 Gew.-%.

Der Gehalt an hydrophilem Modifier beträgt vorzugsweise 0,1 bis 15, insbesondere 1 bis 10 Gew.-%, bezogen auf das Gesamtgewicht der Komponenten (A) und (B).

Die Mengenverhältnisse der Komponenten (A) und (B) werden vorzugsweise so gewählt, daß sie schnell und leicht dosiert und vermischt werden können. Beispielsweise ist das Mengenverhältnis 1:5 bis 5:1 sehr vorteilhaft.

Bei der Herstellung des erfindungsgemäß verwendeten edelmetallkatalysatorfreien hydrophilen Modifiers der allgemeinen Formel (I) geht man vorzugsweise von einem Silan der allgemeinen Formel III

$$R^1RSiZ_2 \qquad (III)$$

aus, in der $R^1$, R und Z die in Formel (I) angegebenen Bedeutungen aufweisen, das vor der Umsetzung destilliert wird, um den Edelmetallkatalysator zu entfernen, der bei der Herstellung des Silans benötigt wird. Das Silan der allgemeinen Formel III ist dazu flüchtig genug. Vorzugsweise weist jedoch x Werte von 3 bis 10 auf und R bedeutet Methyl-oder Ethylreste.

Durch die Destillation werden auch Si-H-Gruppen- oder doppelbindungshaltige Ausgangsverbindungen und Nebenprodukte aus dem Silan III entfernt.

In den nachstehenden allgemeinen Formeln III, IV, V und VI weisen $R^1$, R und Z die in Formel (I) angegebenen Bedeutungen auf, j bedeutet eine ganze Zahl von 2 bis 2000 und k bedeutet eine ganze Zahl von 3 bis 6.

Der erfindungsgemäß verwendete hydrophile Modifier kann durch Silan-Cohydrolyse hergestellt werden, wie in der DE-A-1 519 412 beschrieben (vgl. hierzu auch W.Noll, "Chemie und Technologie der Silicone", Verlag Chemie, Weinheim, 2. Auflage, 1968, Seite 163ff.). Den hydrophilen Modifier, der siliciumgebundene Reste mit aliphatischen Doppelbindungen enthält, stellt man vorzugsweise in an sich bekannter Weise durch Mischhydrolyse oder Mischkondensation eines monomeren, destillierten Silans der allgemeinen Formel III mit anderen hydrolysierbaren Silanen her, die siliciumgebundene Reste mit aliphatischen Doppelbindungen enthalten und Silanen der allgemeinen Formel IV

$$R_2SiZ_2 \qquad (IV)$$

und/oder Siloxanen der allgemeinen Formel V

$$HO(SiR_2O)_jH \qquad (V)$$

und/oder cyclischen Siloxanen der allgemeinen Formel VI

$$(SiR_2O)_k. \qquad (VI)$$

Den hydrophilen Modifier, der Si-H-Gruppen enthält, stellt man vorzugsweise in an sich bekannter Weise durch Mischhydrolyse oder Mischkondensation eines monomeren, destillierten Silans der allgemeinen Formel III mit anderen hydrolysierbaren Silanen her, die siliciumgebundenen Wasserstoff enthalten und Silanen der allgemeinen Formeln IV und gegebenenfalls V und VI.

Den hydrophilen Modifier, der Si-H-Gruppen und zusätzlich Reste mit aliphatischen Doppelbindungen enthält, stellt man vorzugsweise in an sich bekannter Weise durch Mischhydrolyse oder Mischkondensation eines monomeren, destillierten Silans der allgemeinen Formel III mit anderen hydrolysierbaren Silanen her, die siliciumgebundene Wasserstoffatome enthalten, anderen hydrolysierbaren Silanen, die siliciumgebundene Reste mit aliphatischen Doppelbindungen enthalten und Silanen der allgemeinen Formeln IV und gegebenenfalls V und VI.

Die Mischkondensation oder Mischhydrolyse zur Herstellung des hydrophilen Modifiers kann in Gegenwart eines sauren Kondensationskatalysators durchgeführt werden. Dabei werden als saure Kondensationskatalysatoren Phosphornitridchloride bevorzugt. Diese als solche oder in Form ihrer Reaktionsprodukte verwendeten Verbindungen sind alle im wesentlichen aus Phosphor- Stickstoff- und Chloratomen aufgebaut. Sie werden auch als Phosphornitriddichlorid, Phosphornitridchlorid und Phosphornitrilchlorid bezeichnet. Diesen Verbindungen werden unter anderem die Formeln $(PNCl_2)_x$, $Cl_3PNPCl_2NPCl_3 \cdot PCl_6$ und $Cl_3PNPCl_3 \cdot PCl_6$ zugeschrieben.

Vorzugsweise werden im Verfahren zur Herstellung des hydrophilen Modifiers Phosphornitridchloride verwendet und zwar insbesondere solche, die durch Umsetzung von Phosphorpentachlorid mit Ammoniumchlorid zugänglich sind. Insbesondere handelt es sich hierbei um Phosphornitridchloride, welche durch Umsetzung von 400 Gewichtsteilen Phosphorpentachlorid mit 130 Gewichtsteilen Ammoniumchlorid (gemäß Berichte der deutschen chemischen Gesellschaft, 57. Jahrgang, 1924, Seite 1345) und/oder durch Umsetzung von zwei Mol Phosphorpentachlorid mit einem Mol Ammoniumchlorid (gemäß US-A-3 839 388) erhältlich sind. Bevorzugt sind Phosphornitridchloride der letztgenannten Art.

Der saure Kondensationskatalysator wird vorzugsweise als Lösung in einem der nachstehend aufgeführten Lösungsmittel zugegeben.

Die Herstellung des erfindungsgemäßen hydrophilen Modifiers kann in Anwesenheit oder in Abwesenheit von Lösungsmitteln durchgeführt werden. Falls Lösungsmittel verwendet werden, sind Lösungsmittel oder Lösungsmittelgemische mit einem Siedepunkt bzw. Siedebereich von bis zu 120° C bei 0,1 MPa bevorzugt. Beispiele für solche Lösungsmittel sind Ether, wie Dioxan, Tetrahydrofuran, Diethylether, Diethylenglycoldimethylether; chlorierte Kohlenwasserstoffe, wie Dichlormethan, Trichlormethan, Tetrachlormethan, 1,2-Dichlorethan, 1,2,3-Trichlorpropan, Trichlorethylen; Kohlenwasserstoffe, wie Pentan, n-Hexan, Hexan-Isomerengemische, Heptan, Oktan, Waschbenzin, Petrolether, Benzol, Toluol, Xylole; oder Gemische dieser Lösungsmittel.

Die Bezeichnung Lösungsmittel bedeutet nicht, daß sich alle Reaktionskomponenten in diesem lösen müssen. Die Reaktion kann auch in einer Suspension oder Emulsion eines oder mehrerer Reaktionspartner durchgeführt werden . Die Reaktion kann auch in einem Lösungsmittelgemisch mit einer Mischungslücke ausgeführt werden, wobei in jeder der Mischphasen jeweils mindestens ein Reaktionspartner löslich ist.

Besonders bevorzugt ist die Verwendung der in der DE-A-3 903 137(J.Schuster et al.; offengelegt am 16.8.1990 für Wacker-Chemie GmbH, DE) beschriebenen Zusammensetzung aus Phosphornitridchlorid, Tensid als Lösungsvermittler und halogenfreiem Lösungsmittel.

Die erfindungsgemäßen lagerstabilen Polysiloxanmassen sind als dentale Abformmassen zur exakten Abbildung der Oberfläche in der Mundhöhle und zur Doublierung zahntechnischer Gebißmodelle verwendbar. Sie können auch für Abdrücke anderer Körperteile, wie des Gehörgangs, und für alle Zwecke verwendet werden, für die Siliconelastomere mit hydrophilen Eigenschaften vorteilhaft sind, beispielsweise für Kontaktlinsen, Prothesen oder Implantate und nichtmedizinische Zwecke.

In den nachfolgenden Beispielen sind, falls jeweils nicht anders angegeben,
a) alle Mengenangaben auf das Gewicht bezogen;
b) alle Drücke 0,10 MPa (abs.);
c) alle Temperaturen 25° C .
d) Me bedeutet Methylrest
e) Vinylpolymer 1 bedeutet Polydimethlysiloxan mit $\alpha,\omega$-Vinylgruppen mit einer Viskosität von etwa 7000 mm$^2$/s nach Brookfield bei 25° C.
f) Vernetzer bedeutet wasserfreies Dimethylhydrogensiloxaneinheiten als endständige Einheiten aufweisendes Diorganopolysiloxan aus Dimethylsiloxaneinheiten und Methylhydrogensiloxaneinheiten, worin 10 Dimethylsiloxaneinheiten je Methylhydrogensiloxaneinheit vorliegen, mit einer Viskosität von 150 mPas bei 25° C;
g) Katalysator bedeutet einen Pt-1,3-Di-Vinyl-1,1,3,3-tetramethylsiloxanyl-Komplex
h) Inhibitor bedeutet ein Dimethylsiloxan mit durchschnittlich 5 bis 10 Dimethylsiloxaneinheiten und $\alpha,\omega$-Vinylgruppen.

Beispiele

Beispiel 1: Herstellung eines platinfreien hydrophilen Modifiers (Modifier 1)

a) Silansynthese:
Eine Mischung aus 1000 g eines Polyglycolethers der mittleren Formel:

$$H_2C = CHCH_2(OC_2H_4)_4OMe$$

Polyglycol AM 250 (von Hoechst AG)
und 14,6 g einer 1 %igen Lösung von Bis(1,1,3,3-tetramethyl-1,3-divinyldisiloxan)platin-Komplex in Hexan ( =100 ppm Pt) wurde mit 460 g Dichlormethylsilan bei 70° C zur Reaktion gebracht. Nach Abdestillieren der flüchtigen Bestandteile bei 50° C/4 mbar verblieben 1326 g eines gelblichen Öls als Rückstand, das mit 1440 g Isopropanol umgesetzt wurde. Destillation bis 200° C/2 mbar lieferte 646 g einer gelblichen Flüssigkeit der mittleren Formel:

$$H_3C((CH_3)_2CHO)_2Si(CH_2)_3(OC_2H_4)_4OMe \qquad \text{Silan 1}$$

b) Äquilibrierung (Mischkondensation):

153 g eines vinyldimethylendständigen Polydimethylsiloxans mit einer Kettenlänge von ca. 220 $Me_2SiO$-Einheiten (Viskosität 1000 mPas; von Wacker-Chemie GmbH) und 2,6 g Dimethylvinylchlorsilan wurden bei Raumtemperatur mit 0,54 g einer 25 %-igen Lösung von Phosphornitrilchlorid ("$PNCl_2$") in 1,2,3-Trichlorpropan versetzt und unter Rühren auf 100 °C aufgeheizt. Binnen 1,5 Stunden wurden 116 g des gemäß a) hergestellten Silans 1 bei 100 °C unter Rühren zudosiert. Anschließend rührte man eine Stunde bei 100 °C. Nach Hydrolyse mit 63 g Wasser wurden flüchtige Bestandteile abdestilliert. Als Rückstand verblieben 210 g eines rotbraunen, klaren Öls mit einer Viskosität von 100 $mm^2/s$, das laut Elementaranalyse (Röntgenfluoreszenz) keine nachweisbaren Mengen an Platin enthielt.

Die mittlere Formel des so erhaltenen hydrophilen Modifiers 1 lautete nach $^{29}$Si- und $^1$H-NMR-Spektren

$$[H_2C=CH\text{-}SiMe_2O_{1/2}]_{1,88}[SiMe_2OiPr]_{0,12}[SiMe_2O_{2/2}]_{44}\ [SiR^1MeO_{2/2}]_{7,5} \text{ mit } R^1 = (CH_2)_3(OC_2H_4)_4OMe$$

Beispiel 2: Herstellung eines platinfreien hydrophilen Modifiers (Modifier 2)

a) Silansynthese:

2688 g eines Polyethers der mittleren Formel:

$$H_2C=CHCH_2\ (OC_2H_4)_6OMe$$

Arlypon® AL 6 (methyliert) (von Hoechst AG)

wurden in Gegenwart von 0,67 g Platintetrachlorid (= 100 ppm Pt) mit 1072 g Diethoxymethylsilan bei 70 °C umgesetzt.

Eine Destillation bis 200 °C/2 mbar lieferte 2624 g eines Silans der mittleren Formel:

$$H_3C((CH_3CH_2O)_2Si(CH_2)_3(OC_2H_4)_6OMe \qquad \text{Silan 2}$$

b) Äquilibrierung:

341 g Octamethylcyclotetrasiloxan, 12,3 g Chlordimethylvinylsilan, und 262 g Silan 2 wurden in Gegenwart von 2,2 ml einer 25 %igen Lösung von $PNCl_2$ in 1,2,3-Trichlorpropan bei 80 °C äquilibriert. Nach Zugabe von 139 g Wasser wurden bis 120 °C bei Normaldruck alle flüchtigen Bestandteile abdestilliert. Man erhielt als Rückstand 476 g eines Öles mit einer Viskosität von 200 $mm^2/s$. Die mittlere Formel des so erhaltenen hydrophilen Modifiers 2 lautete nach $^{29}$Si- und $^1$H-NMR-Spektren:

$$[H_2C=CH\text{-}SiMe_2O_{1/2}]_2[SiMe_2O_{2/2}]_{39}[SiR^1MeO_{2/2}]_{12} \text{ mit } R^1 = (CH_2)_3(OC_2H_4)_6OMe$$

Beispiel 3: Herstellung eines platinhaltigen hydrophilen Modifiers (Modifier 3)

Eine Lösung von 336 g eines Polyethers der mittleren Formel:

$$H_2C=CHCH_2(OC_2H_4)_6OMe$$

Arlypon® AL 6 (methyliert) (von Hoechst AG)

in 50 ml Toluol wurde in Gegenwart von 145 mg Hexachloroplatinsäure mit 1115 g eines Polysiloxans der mittleren Formel:

$$[Me_3SiO_{2/2}]_2[SiMe_2O_{2/2}]_{51}[SiHMeO_{2/2}]_9$$

bei 80 °C umgesetzt. Nach Abziehen der leichtflüchtigen Bestandteile im Vakuum und Filtration über 30 g Aktivkohle und 40 g Kieselgel verblieben 1414 g eines rotbraunen Öls, dem nach $^1$H- und $^{29}$Si-NMR-Spektren die folgende mittlere Formel zugeordnet werden konnte:

$$[Me_3SiO_{2/2}]_2[SiMe_2O_{2/2}]_{51}[SiR^1MeO_{2/2}]_9 \text{ mit } R^1 = (CH_2)_3(OC_2H_4)_6OMe$$

Die Viskosität betrug 210 $mm^2/s$.

Über $^1$H-NMR-Spektroskopie konnte ein Si-H-Restgehalt von 0,35 Mol% ermittelt werden. Laut Elementaranalyse enthielt das Öl 65 ppm Platin.

Beispiel 4: Herstellung einer erfindungsgemäßen Dentalmasse "light body"

Komponente (A)

In einem evakuierbaren Edelstahl-Planetenmischer mit einem Fassungsvermögen von 6 l und einer durchschnittlichen Rührgeschwindigkeit von 150 UpM wurden
- 1890 g Vinylpolymer 1
- 30 g Hydrophil-Modifier 2 (entspricht 1 Gew.-%)
mit
- 840 g Quarzmehl mit einer Korngröße von 1 - 30 $\mu$m (von Quarzwerke Frechen)
- 60 g Aerosil® R 972 (von Degussa AG)
- 120 g Calciniertes Kieselgur mit poröser Struktur Korngröße 1 - 20 $\mu$m (von Ceca S.A.)
- 10 g Chromoxid grün Pigment
- 6 g TiO$_2$ Pigment

vermischt und innerhalb von 60 min. zu einer homogenen, selbstnivellierenden Masse verarbeitet.
Anschließend wurden noch
- 36 g Katalysator
- 3 g Inhibitor

zugegeben. Die Gesamtmischung wurde noch weitere 15 min. bei vermindertem Druck von 50 - 100 mbar gerührt.

Komponente B

In einem evakuierbaren Edelstahl-Planetenmischer mit einem Fassungsvermögen von 6 l und einer durchschnittlichen Rührgeschwindigkeit von 150 UpM wurden
- 1250 g Vinylpolymer 1
- 150 g Hydrophil-Modifier 2 (entspricht 4,91 Gew.-%)
- 450 g Vernetzer
mit
- 960 g Quarzmehl mit einer Korngröße 1 - 30 $\mu$m (von Quarzwerke Frechen)
- 120 g Aerosil® R 972 (von Degussa AG)
- 90 g Calciniertes Kieselgur mit poröser Struktur, Korngröße 1 - 20 $\mu$m (von Ceca S.A.)
- 36 g Chromoxidgrün Pigment

vermischt und innerhalb 60 min zu einer homogenen selbstnivellierenden Masse verarbeitet.
Anschließend wurden noch 15 min. bei vermindertem Druck zwischen 50 - 100 mbar nachgerührt.

Beispiel 5: Herstellung einer erfindungsgemäßen Dentalmasse "medium body"

Komponente A

In einem evakuierbaren Edelstahl-Planetenmischer mit einem Fassungsvermögen von 6 l und einer durchschnittlichen Rührgeschwindigkeit von 150 UpM wurden
- 1530 g Vinylpolymer 1
- 30 g Hydrophil-Modifier 2
- 1260 g Quarzmehl mit einer Korngröße von 1 - 30 $\mu$m (von Quarzwerke Frechen)
- 60 g Aerosil® R 972 (von Degussa AG)
- 108 g Calciniertes Kieselgur mit poröser Struktur, Korngröße 1 - 20 $\mu$m (von Ceca S.A.)
- 0,15 g Co/Al - Spinell - Pigment

vermischt und innerhalb 60 min zu einer homogenen pastösen Masse verarbeitet. Anschließend wurden noch
- 36 g Katalysator
- 3 g Inhibitor

zugegeben. Die Gesamtmischung wurde noch weitere 15 min. bei vermindertem Druck von 50 - 100 mbar gerührt.

Komponente B

In einem evakuierbaren Edelstahl-Planetenmischer mit einem Fassungsvermögen von 6 l und einer durchscnittlichen Rührgeschwindigkeit von 150 UpM wurden
- 1200 g Vinylpolymer 1
- 150 g Hydrophil-Modifier 2
- 360 g Vernetzer
- 1020 g Quarzmehl mit einer Korngröße 1 - 30 $\mu$m (von Quarzwerke Frechen)
- 150 g Aerosil® R 972 (von Degussa AG)
- 90 g Calciniertes Kieselgur mit poröser Struktur, Korngröße 1 - 20 $\mu$m (von Ceca S.A.)
- 6 g $TiO_2$ Pigment
- 40 g Co/Al - Spinellpigment

vermischt und innerhalb 60 min. zu einer homogenen pastösen Masse verarbeitet. Anschließend wurde 15 min. bei vermindertem Druck von 50 - 100 mbar gerührt.

Beispiel 6 (Vergleichsbeispiel)

Die Komponenten A und B wurden wie in Beispiel 4 hergestellt, jedoch wurde statt des platinfreien hydrophilen Modifiers 2 der platinhaltige hydrophile Modifier 3 verwendet.

Beispiel 7 (Vergleichsbeispiel)

Die Komponenten A und B wurden wie in Beispiel 5 hergestellt, jedoch wurde statt des platinfreien hydrophilen Modifiers 2 der platinhaltige hydrophile Modifier 3 verwendet.

Beispiel 8 (Vergleichsbeispiel)

In Beispiel 5 wurden zur Komponente B 5 ppm Pt in Form von Hexachloro-Platinsäure (= 0,05 g $H_2PtCl_6$) zugegeben.

Beispiel 9

Die nachstehend aufgeführten Untersuchungsergebnisse erbringen den experimentellen Nachweis für die Überlegenheit der erfindungsgemäßen Abformpräparate I und II im Vergleich zu Präparaten entsprechend dem Stand der Technik (Abformpräparate III und IV) im Hinblick auf die Veränderung der Benetzungseigenschaften dieser Abformpräparate im Kontakt mit wäßrigen Systemen (Sterilisation).

Die Siliconabformpräparate I, II, III und IV wurden im vorgeschriebenen Mischungsverhältnis der Komponenten (A):(B) = 1:1 homogen miteinander vermischt, anschließend in zylindrische Edelstahlformen (Höhe: 6 mm, Durchmesser: 35 mm) eingefüllt, so daß sie bei Raumtemperatur nach wenigen Minuten als zylindrische Vulkanisate entformt werden konnten.

Die Vulkanisatproben der Präparate I, II, III und IV wurden senkrecht zur Kreisfläche entlang ihres Durchmessers halbiert, mit einem Tropfen destillierten Wassers auf der Oberfläche versehen und hinsichtlich der Wasserkontaktwinkel im zeitlichen Abstand von 30 Sek. und 3 Min. nach Aufbringen des Wassertropfens mit Hilfe eines Mikroskopgoniometers vermessen. Anschließend wurden die Wassertropfen von der Vulkanisatoberfläche mit etwas Zellstoff abgesaugt, und die Vulkanisatproben I, II, III und IV vergleichenden Sterilisationsbedingungen unterworfen (Lagerung bei Raumtemperatur in einer 1,37 %-igen wässrigen $KClO_3$-Lösung für 6 Stunden). Nach der Sterilisation wurden die Vulkanisatproben erneut mit Zellstoff getrocknet, jeweils auf ihrer Oberfläche mit einem Tropfen destillierten Wassers versetzt und erneut einer vergleichenden Messung der Wasserkontaktwinkel mit einem Mikroskopgoniometer unterzogen.

Die Ergebnisse dieser Untersuchung sind als Mittelwerte von jeweils 6 Probekörperhälften in der nachfolgenden Tabelle I zusammengestellt und belegen, daß die Präparate I und II aus den erfindungsgemäßen lagerstabilen Polysiloxanmassen gemäß der Beispiele 4 bzw. 5 im Gegensatz zu den nicht erfindungsgemäßen Präparaten III und IV nach der Sterilisation mit wässrigen Medien keinerlei Extraktionserscheinungen zeigen.

Das Mikroskogoniometer trägt die Bezeichnung NRL C.A. Goniometer, Modell No. 100-230 der Firma Ramé-Hart Inc., USA.

Tabelle I

| Präparat | Viskosität gemäß ADA 19[1] | Meßwerte vor Sterilisation Wasserkontaktwinkel | | Meßwerte nach Sterilisation Wasserkontaktwinkel | |
|---|---|---|---|---|---|
| | | 30 sec | 3 min | 30 sec | 3 min |
| I erfindungsgemäß Beispiel 4 | Low | 66 ° | 46 ° | 66 ° | 47 ° |
| II erfindungsgemäß Beispiel 5 | Medium | 63 ° | 45 ° | 63 ° | 45 ° |
| 2 nicht erfindungsgemäß | Low | 64 ° | 44 ° | 63 ° | 62 ° |
| 3 nicht erfindungsgemäß | Medium | 100 ° | 61 ° | 103 ° | 75 ° |

1    American Dental Association, Spezifikation No. 19

2    III: Express; Light Body Regular Set; von 3M Company

3    IV:  Unosil S; von De Trey Dentsply GmbH

Beispiel 10: Untersuchung der Lagerstabilität der Dentalmassen aus Beispielen 4 bis 8

Die Ergebnisse sind jeweils in Tabelle II aufgeführt.

a) Messung der Konsistenz gemäß ADA 19

Die Komponenten A und B und die 1:1-Mischungen der Komponenten A und B wurden 1 Tag bei Raumtemperatur (RT) bzw. 7 Tage bei 70°C gelagert und anschließend gemäß der Spezifikation no. 19 der American Dental Association (ADA 19) untersucht:

Ein Volumen von 0,50 ml Mischung wurde auf eine mit Polyäthylen oder Cellophan bedeckte Glasplatte gegeben. Nach 1 1/2 Minuten werden 0,5 ml Material mit einem Polyäthylen- oder Cellophanplättchen, sowie einer Glasplatte von 75 ± 5 g plus 500 g Gewicht bedeckt. 12 Minuten nach Mischbeginn wird die Belastung (Glasplatte und 500 g Gewicht) entfernt und der größte und kleinste Durchmesser gemessen. Der Wert für die Konsistenz wird aus dem Durchschnitt von drei Bestimmungen errechnet und auf den nächsten Millimeter auf- oder abgerundet.

b) Messung der Topfzeit

Die Komponenten wurden im Verhältnis 1:1 im konischen Topfzeit-Becher (TZ-Becher) mit einem Spatel vermischt. Die Zeitnahme erfolgte gleichzeitig mit dem Mischbeginn. Der Mischvorgang erfolgte gleichmäßg und intensiv und dauerte 45 sec. Nach dem Mischen wurde die Mischbewegung langsam fortgesetzt.

Die TZ war erreicht, wenn eine Verarbeitung des Produkts nicht mehr möglich war.

c) Messung des Wasserkontaktwinkels

Die Komponenten A und B wurden gemäß Beispiel 9 im Verhältnis 1:1 vulkanisiert und untersucht.

d) Shorehärte der vulkanisierten Abformpräparate

Die 1:1-Mischungen wurden 15 min. bei RT ausvulkanisiert und danach gemäß DIN 53505 untersucht.

e) Visuelle Beurteilung der Dentalmassen

Die Komponenten A und B wurden im Verhältnis 1:1 45 sec. vermischt und danach visuell beurteilt.

Tabelle II

| | Beispiel 4 | | Beispiel 5 | | Beispiel 6 * | | Beispiel 7 * | | Beispiel 8 * | |
|---|---|---|---|---|---|---|---|---|---|---|
| Konsistenz (mm) nach ADA 19 | RT | 7d/70°C | RT | 7d/70°C | RT | 7d/70°C | RT | 7d/70°C | RT | 7d/70°C |
| a) Komponente A | 45 | 43 | 44 | 43 | 45 | 37 | 44 | 35 | 44 | 44 |
| Komponente B | 45 | 46 | 45 | 44 | 45 | 1) | 45 | 2) | 45 | 3) |
| Komponente A+B | 39 | 38 | 34 | 32 | 39 | | 34 | | 33 | |
| b) Topfzeit (sec.) | 210 | 205 | 210 | 200 | 200 | | 210 | | 200 | |
| c) Wasserkontakt- winkel nach 3 min. | 46° | 46° | 45° | 45° | 46° | | 45° | | 45° | |
| d) Shorehärte nach 15 min., DIN 53505 | 38 | 40 | 51 | 52 | 38 | | 51 | | 51 | |
| e) Beurteilung visuell | homogen, selbst- nivell. Masse | wie bei RT | homogen pastöse Masse | wie bei RT | homogen selbst- nivell. Masse | 4) | homogen pastöse Masse | 5) | homogen pastöse Masse | zum größ- ten Teil vulkani- sierte Masse |

* Vergleichsbeispiele

1), 2), 3)   nicht meßbar, da Vulkanisatanteile vorlagen

4), 5)   zum größten Teil vulkanisierte Masse

7d/70°C = Lagerung der Komponenten 7 Tage bei 70°C im Trockenschrank

## Patentansprüche

1. Lagerstabile, permanent wasserbenetzbare elastomere Vulkanisate ergebende Polysiloxanmasse, bestehend aus den getrennt gelagerten Komponenten

(A), die eine Masse aus Polysiloxanen, welche SiC-gebundene $C_1$-$C_6$-Alkylreste und/oder Phenylreste aufweisen und mindestens 2 $C_1$-$C_6$-Alkenylreste pro Molekül besitzen, und einen Edelmetallkatalysator umfaßt, wobei die Komponente (A) im wesentlichen frei von Si-H-Gruppen ist und

(B), die eine edelmetallkatalysatorfreie Masse aus Polysiloxanen, welche SiC-gebundene $C_1$-$C_6$-Alkylreste und/oder Phenylreste aufweisen und mindestens 3 Si-H-Gruppen pro Molekül besitzen, und gegebenenfalls zusätzlich aus Polysiloxanen, welche SiC-gebundene $C_1$-$C_6$-Alkylreste und/oder Phenylreste aufweisen und mindestens 2 $C_1$-$C_6$-Alkenylreste pro Molekül besitzen umfaßt, wobei ein hydrophiler Modifier der allgemeinen Formel I

$$[R_3SiO_{1/2}]_a[R^1R_2SiO_{1/2}]_b[ZR_2SiO_{1/2}]_c[ZRR^1SiO_{1/2}]_d \qquad [RR^1SiO_{2/2}]_e[R_2SiO_{2/2}]_f[SiO_{4/2}]_g[RSiO_{3/2}]_h-[R^1SiO_{3/2}]_i, \qquad (I)$$

in der die Reste

**R** Wasserstoffatome oder gleiche oder verschiedene einwertige, gegebenenfalls halogensubstituierte über SiC-gebundene $C_1$-$C_{12}$-Kohlenwasserstoffreste bedeuten, wobei mindestens einer der Reste R eine aliphatische Doppelbindung aufweist oder ein Wasserstoffatom bedeutet;

**$R^1$** die allgemeine Formel II

$$E[OY]_xR^2 \qquad (II)$$

bedeutet, in der **E** eine Einfachbindung oder einen $C_1$-$C_6$-Alkylenrest, **Y** gleiche oder verschiedene $C_1$-$C_4$-Alkylenreste bedeutet, **$R^2$** eine Hydroxylgruppe, einen $C_1$-$C_6$-Alkoxy- oder $C_1$-$C_6$-Oxycarbonylalkylrest bedeutet und **x** die Werte 1 bis 20 hat;

**Z** die Bedeutungen von $R^2$ aufweist oder ein Halogenatom bedeutet;

mit der Maßgabe, daß a, b, c und d jeweils unabhängig voneinander Werte von 0 bis 8 haben, die Summe von $a+b+c+d$ 2 bis 8 beträgt, die Summe von $a+b+c+d+e+f+g+h+i$ 10 bis 400 beträgt und das Verhältnis der Summen $a+c+f+g+h : b+d+e+i$ 100 : 1 bis 1 : 1 beträgt,

welcher

    i) Reste mit aliphatischen Doppelbindungen aufweist, in der Komponente (A) und (B) enthalten ist und/oder

    ii) Si-H-Gruppen und/oder Reste mit aliphatischen Doppelbindungen aufweist, in der Komponente (B) enthalten ist,

mit der Maßgabe, daß wenn der hydrophile Modifier Si-H-Gruppen aufweist oder in eine Si-H-Gruppen-haltige Komponente (B) gemischt wird, $c+d < 0,02 (a+b+c+d+e+f+g+h+i)$ ist,

**dadurch gekennzeichnet**, daß der hydrophile Modifier frei von Edelmetallkatalysator ist.

2. Lagerstabile Polysiloxanmasse nach Anspruch 1, wobei der hydrophile Modifier Reste mit aliphatischen Doppelbindungen aufweist und sowohl i) in der Komponente (A) als auch ii) in der Komponente (B) enthalten ist.

3. Lagerstabile Polysiloxanmasse nach Anspruch 1 oder 2, wobei das Verhältnis $a+c+f+g+h : x-(b+d+e+i)$ 60:40 bis 90:10 beträgt.

4. Lagerstabile Polysiloxanmasse nach einem der Ansprüche 1 bis 3, wobei x Werte von 3 bis 10 hat.

5. Lagerstabile Polysiloxanmasse nach einem der Ansprüche 1 bis 4, wobei $f > 0,8(f+g+h+i)$, $e > 0,6-(d+e+i)$ und b, c, d, g, h und $i < 0,2(a+b+c+d+e+f+g+h+i)$.

6. Lagerstabile Polysiloxanmasse nach einem der Ansprüche 1 bis 5, wobei $a > 0,5(a+b+c+d)$.

7. Verfahren zur Herstellung einer lagerstabilen Polysiloxanmasse nach einem der Ansprüche 1 - 6, wobei in einem ersten Schritt ein Silan der allgemeinen Formel III

$$R^1RSiZ_2 \qquad (III),$$

in der die Reste

**R** Wasserstoffatome oder gleiche oder verschiedene einwertige, gegebenenfalls halogensubstituierte

EP 0 602 128 B1

über SiC-gebundene $C_1$-$C_{12}$-Kohlenwasserstoffreste bedeuten,
**R¹** die allgemeine Formel II

E[OY]$_x$R² (II)

bedeutet, in der **E** eine Einfachbindung oder einen $C_1$-$C_6$-Alkylenrest, **Y** gleiche oder verschiedene $C_1$-$C_4$-Alkylenreste bedeutet, **R²** eine Hydroxylgruppe, einen $C_1$-$C_6$-Alkoxy- oder $C_1$-$C_6$-Oxycarbonylalkylrest bedeutet und **x** die Werte 1 bis 20 hat und
**Z** die Bedeutungen von R² aufweist oder ein Halogenatom bedeutet;
destilliert wird, um Edelmetallkatalysator zu entfernen, in einem zweiten Schritt das Silan der allgemeinen Formel III zusammen mit anderen hydrolysierbaren Silanen, die siliciumgebundene Wasserstoffatome enthalten und/oder anderen hydrolysierbaren Silanen, die siliciumgebundene Reste mit aliphatischen Doppelbindungen enthalten und Silanen der allgemeinen Formeln IV

$R_2SiZ_2$ (IV)

und/oder Siloxanen der allgemeinen Formel V

$HO(SiR_2O)_jH$ (V)

und/oder cyclischen Siloxanen der allgemeinen Formel VI

$(SiR_2O)_k$, (VI),

wobei in den allgemeinen Formeln IV, V und VI
R und Z die vorstehenden Bedeutungen aufweisen,
j eine ganze Zahl von 2 bis 2000 bedeutet und
k eine ganze Zahl von 3 bis 6 bedeutet,
in an sich bekannter Weise zu dem hydrophilen Modifier hydrolysiert oder kondensiert werden und
in einem dritten Schritt der hydrophile Modifier, der
   i) Reste mit aliphatischen Doppelbindungen aufweist, in die Komponenten (A) und (B) gemischt wird und/oder
   ii) Si-H-Gruppen und/oder Reste mit aliphatischen Doppelbindungen aufweist, in die Komponente (B) gemischt wird.

8. Verwendung der lagerstabilen Polysiloxanmasse nach einem der Ansprüche 1 bis 6 und der nach Anspruch 7 hergestellten lagerstabilen Polysiloxanmasse als dentale Abformmasse.

**Claims**

1. Storage-stable polysiloxane composition which gives permanently water-wettable elastomeric vulcanizates and consists of the separately stored components
   (A) which comprises a composition of polysiloxanes which contain SiC-bonded $C_1$-$C_6$-alkyl radicals and/or phenyl radicals and have at least 2 $C_1$-$C_6$-alkenyl radicals per molecule, and a noble metal catalyst, component (A) being essentially free from Si-H groups, and
   (B) which comprises a composition of polysiloxanes which contain SiC-bonded $C_1$-$C_6$-alkyl radicals and/or phenyl radicals and have at least 3 Si-H groups per molecule, and, if desired, additionally of polysiloxanes which contain SiC-bonded $C_1$-$C_6$-alkyl radicals and/or phenyl radicals and have at least 2 $C_1$-$C_6$-alkenyl radicals per molecule, which composition is free from noble metal catalysts,
   wherein a hydrophilic modifier of the general formula I

$[R_3SiO_{1/2}]_a[R^1R_2SiO_{1/2}]_b[ZR_2SiO_{1/2}]_c[ZRR^1SiO_{1/2}]_d$     $[RR^1SiO_{2/2}]_e[R_2SiO_{2/2}]_f[SiO_{4/2}]_g[RSiO_{3/2}]_h$-$[R^1SiO_{3/2}]_i$ (I)

in which the radicals
**R** denote hydrogen atoms or identical or different monovalent, optionally halogen-substituted $C_1$-$C_{12}$-hydrocarbon radicals bonded via SiC- [sic], in which at least one of the radicals R contains an aliphatic double bond or denotes a hydrogen atom;

$R^1$ denotes the general formula II

$E[OY]_xR^2$     (II)

in which **E** denotes a single bond or a $C_1$-$C_6$-alkylene radical, **Y** denotes identical or different $C_1$-$C_4$-alkylene radicals, $R^2$ denotes a hydroxyl group or a $C_1$-$C_6$-alkoxy or $C_1$-$C_6$-oxycarbonylalkyl radical and x has values from 1 to 20; and

**Z** has the meanings of $R^2$ or denotes a halogen atom;

with the proviso that a, b, c and d in each case independently of one another have values from 0 to 8, the sum of $a+b+c+d$ is 2 to 8, the sum of $a+b+c+d+e+f+g+h+i$ is 10 to 400 and the ratio of the sums of $a+c+f+g+h : b+d+e+i$ is 100 : 1 to 1 : 1,

which modifier

   i) contains radicals having aliphatic double bonds and is present in component (A) and (B), and/or
   ii) contains Si-H groups and/or radicals having aliphatic double bonds and is present in component (B),

with the proviso that, if the hydrophilic modifier contains Si-H groups or is mixed into a component (B) which contains Si-H groups, $c+d$ is $< 0.02 (a+b+c+d+e+f+g+h+i)$,

characterized in that the hydrophilic modifier is free from noble metal catalyst.

2. Storage-stable polysiloxane composition according to Claim 1, wherein the hydrophilic modifier contains radicals having aliphatic double bonds and is contained both i) in component (A) and ii) in component (B).

3. Storage-stable polysiloxane composition according to Claim 1 or 2, wherein the ratio of $a+c+f+g+h : x(b+d+e+i)$ is 60:40 to 90:10.

4. Storage-stable polysiloxane composition according to one of Claims 1 to 3, wherein x has values from 3 to 10.

5. Storage-stable polysiloxane composition according to one of Claims 1 to 4, wherein $f > 0.8(f+g+h+i)$, $e > 0.6(d+e+i)$ and b, c, d, g, h and $i < 0.2(a+b+c+d+e+f+g+h+i)$.

6. Storage-stable polysiloxane composition according to one of Claims 1 to 4, wherein $a > 0.5$-$(a+b+c+d)$.

7. Process for the preparation of a storage-stable polysiloxane composition according to one of Claims 1 - 6, in which in a first step a silane of the general formula III

$R^1RSiZ_2$     (III),

in which the radicals
**R** denote hydrogen atoms or identical or different monovalent, optionally halogen-substituted $C_1$-$C_{12}$-hydrocarbon radicals bonded via SiC-,
$R^1$ denotes the general formula II

$E[OY]_xR^2$     (II)

in which **E** denotes a single bond or a $C_1$-$C_6$-alkylene radical, **Y** denotes identical or different $C_1$-$C_4$-alkylene radicals, $R^2$ denotes a hydroxyl group or a $C_1$-$C_6$-alkoxy or $C_1$-$C_6$-oxycarbonylalkyl radical and **x** has values from 1 to 20; and
**Z** has the meanings of $R^2$ or denotes a halogen atom;
is distilled in order to remove noble metal catalyst, in a second step the silane of the general formula III together with other hydrolysable silanes which contain silicon-bonded hydrogen atoms and/or other hydrolysable silanes which contain silicon-bonded radicals having aliphatic double bonds and silanes of the general formulae [sic] IV

$R_2SiZ_2$     (IV)

and/or siloxanes of the general formula V

$$HO(SiR_2O)_jH \quad (V)$$

and/or cyclic siloxanes of the general formula VI

$$(SiR_2O)_k, \quad (VI),$$

in which, in the general formulae IV, V and VI,
R and Z have the above meanings,
j is an integer from 2 to 2000, and
k is an integer from 3 to 6, are hydrolysed or condensed in a manner known per se to give the hydrophilic modifier, and
in a third step the hydrophilic modifier, which
    i) contains radicals having aliphatic double bonds, is mixed into components (A) and (B), and/or
    ii) contains Si-H groups and/or radicals having aliphatic double bonds, is mixed into component (B).

8. The use of the storage-stable polysiloxane composition according to one of Claims 1 to 6 and of the storage-stable polysiloxane composition prepared according to Claim 7 as a dental impression composition.

## Revendications

1. Composition de polysiloxanes stable au stockage donnant des produits vulcanisés élastomères mouillables à l'eau en permanence, composée des constituants suivants, conservés séparément:

le constituant (A), qui comprend une composition de polysiloxanes contenant des restes alkyle en $C_1$-$C_6$ et/ou des restes phényle liés par des liaisons SiC et possédant au moins 2 restes alcényle en $C_1$-$C_6$ par molécule, et un catalyseur à base de métaux nobles, le constituant (A) étant essentiellement exempt de groupes Si-H, et

le constituant (B), qui comprend une composition sans catalyseur à base de métaux nobles constituée de polysiloxanes contenant des restes alkyle en $C_1$-$C_6$ et/ou des restes phényle liés par des liaisons SiC et possédant au moins 3 groupes Si-H par molécule, et éventuellement en plus de polysiloxanes contenant des restes alkyle en $C_1$-$C_6$ et/ou des restes phényle liés par des liaisons SiC et possédant au moins 2 restes alcényle en $C_1$-$C_6$ par molécule,

et dans laquelle un agent modifiant hydrophile de formule générale I

$$[R_3SiO_{1/2}]_a[R^1R_2SiO_{1/2}]_b[ZR_2SiO_{1/2}]_c[ZRR^1SiO_{1/2}]_d[RR^1SiO_{2/2}]_e[R_2SiO_{2/2}]_f[SiO_{4/2}]_g[ \quad\quad\quad RSiO_{3/2}]_h\text{-}[R^1SiO_{3/2}]_i \quad (I)$$

dans laquelle

les restes R représentent des atomes d'hydrogène ou des restes hydrocarbonés en $C_1$-$C_{12}$ monovalents identiques ou différents, éventuellement halogénés, liés par des liaisons SiC, au moins l'un des restes R contenant une double liaison aliphatique ou représentant un atome d'hydrogène;

$R^1$ représente la formule générale II

$$E[OY]_xR^2 \quad (II)$$

dans laquelle E est une liaison simple ou un reste alkylène en $C_1$-$C_6$, Y représente des restes alkylène en $C_1$-$C_4$ identiques ou différents, $R^2$ représente un groupe hydroxy ou un reste alcoxy en $C_1$-$C_6$ ou oxycarbonylalkyle en $C_1$-$C_6$, et

x a une valeur comprise entre 1 et 20;

Z a la signification de $R^2$ ou représente un atome d'halogène;

sous réserve que a, b, c, et d aient indépendamment les uns des autres des valeurs de 0 à 8, que la somme de $a+b+c+d$ soit comprise entre 2 et 8, que la somme de $a+b+c+d+e+f+g+h+i$ soit comprise entre 10 et 400 et que le rapport des sommes $a+c+f+g+h : b+d+e+i$ soit compris entre 100 : 1 et 1 : 1,

    i) qui contient des restes à doubles liaisons aliphatiques, est contenu dans les constituants (A) et (B) et/ou

EP 0 602 128 B1

ii) qui contient des groupes Si-H et/ou des restes à doubles liaisons aliphatiques, est contenu dans le constituant (B),

sous réserve que, lorsque l'agent modifiant hydrophile contient des groupes Si-H ou est mélangé avec un constituant (B) contenant des groupes Si-H, on ait $c + d < 0,02 (a + b + c + d + e + f + g + h + i)$,

caractérisée en ce que l'agent modifiant hydrophile ne contient pas de catalyseur à base de métaux nobles.

2. Composition de polysiloxanes stable au stockage selon la revendication 1, dans laquelle l'agent modifiant hydrophile contient des restes ayant des doubles liaisons aliphatiques et est contenu aussi bien i) dans le constituant (A) que ii) dans le constituant (B).

3. Composition de polysiloxanes stable au stockage selon la revendication 1 ou 2, dans laquelle le rapport $a + c + f + g + h : x(b + d + e + i)$ est compris entre 60:40 et 90:10.

4. Composition de polysiloxanes stable au stockage selon l'une des revendications 1 à 3, dans laquelle x a des valeurs de 3 à 10.

5. Composition de polysiloxanes stable au stockage selon l'une des revendications 1 à 4, dans laquelle on a $f > 0,8(f + g + h + i)$, $e > 0,6(d + e + i)$ et b, c, d, g, h et $i < 0,2(a + b + c + d + e + f + g + h + i)$.

6. Composition de polysiloxanes stable au stockage selon l'une des revendications 1 à 5, dans laquelle a est $> 0,5 (a + b + c + d)$.

7. Procédé de préparation d'une composition de polysiloxanes stable au stockage selon l'une des revendications 1 à 6, dans lequel, dans une première étape, on distille un silane de formule générale III

$R^1RSiZ_2$     (III),

dans laquelle les restes R représentent des atomes d'hydrogène ou des restes hydrocarbonés en $C_1$-$C_{12}$ monovalents identiques ou différents, éventuellement halogénés, liés par des liaisons SiC, $R^1$ représente la formule générale II

$E[OY]_xR^2$     (II)

dans laquelle E est une liaison simple ou un reste alkylène en $C_1$-$C_6$, Y représente des restes alkylène en $C_1$-$C_4$ identiques ou différents, $R^2$ représente un groupe hydroxy ou un reste alcoxy en $C_1$-$C_6$ ou oxycarbonylalkyle en $C_1$-$C_6$, et x a une valeur comprise entre 1 et 20, et Z a la signification de $R^2$ ou représente un atome d'halogène; pour éliminer le catalyseur à base de métaux nobles, dans une seconde étape, on hydrolyse ou on condense de façon connue en soi le silane de formule générale III avec d'autres silanes hydrolysables qui contiennent des atomes d'hydrogènes liés au silicium et/ou avec d'autres silanes hydrolysables qui contiennent des restes à doubles liaisons aliphatiques liés au silicium et avec des silanes de formule générale IV

$R_2SiZ_2$     (IV)

et/ou des siloxanes de formule générale V

$HO(SiR_2O)_jH$     (V)

et/ou des siloxanes cycliques de formule générale VI

$(SiR_2O)_k$     (VI),

où, dans les formules générales IV, V et VI,
R et Z ont les significations précédentes,
j est un nombre entier de 2 à 2000 et
k est un nombre entier de 3 à 6,

19

pour donner l'agent modifiant hydrophile, et

dans une troisième étape,

i) on mélange l'agent modifiant hydrophile qui contient des doubles liaisons aliphatiques dans les constituants (A) et (B) et/ou

ii) on mélange l'agent modifiant hydrophile qui contient des groupes Si-H et/ou des restes à doubles liaisons aliphatiques, dans le constituant (B).

8. Utilisation de la composition de polysiloxanes stable au stockage selon l'une des revendications 1 à 6 et de la composition de polysiloxanes stable au stockage préparée selon la revendication 7 comme composition de moulage dentaire.